# EUROPEAN PATENT SPECIFICATION

(11) **EP 1 527 172 B1**
(45) Date of publication and mention of the grant of the patent: **26.11.2008**
(21) Application number: 03752746.2
(22) Date of filing: 15.05.2003
(51) Int. Cl.: C12N 15/10, C07H 1/06, C12Q 1/68

(54) **METHOD FOR NUCLEIC ACID EXTRACTION AND NUCLEIC ACID PURIFICATION**
METHODE FÜR DIE EXTRAKTION UND AUFREINIGUNG VON NUKLEINSÄUREN
PROCEDE D'EXTRACTION ET DE PURIFICATION D'ACIDES NUCLEIQUES

(30) Priority: 17.05.2002 DE 10222133; 17.05.2002 DE 20207793 U; 26.06.2002 US 186166
(43) Date of publication of application: 04.05.2005
(73) Proprietor: Molzym GmbH & Co. KG, 28359 Bremen (DE)
(72) Inventor: LORENZ, Michael, G., 26160 Bad Zwischenahn (DE)
(74) Representative: UEXKÜLL & STOLBERG
(86) International application number: PCT/EP2003/005117
(87) International publication number: WO 2003/097831

(56) References cited:
- WO-A-92/00983
- WO-A-95/02049
- WO-A-99/58664
- US-A1- 2002 010 145
- KHANNA M ET AL: "Transformation of Bacillus subtilis by DNA bound on montmorillonite and effect of DNase on the transforming ability of bound DNA" APPLIED AND ENVIRONMENTAL MICROBIOLOGY, WASHINGTON,DC, US, vol. 58, no. 6, June 1992 (1992-06), pages 1930-1939, XP002111372 ISSN: 0099-2240
- KOO KAI ET AL: "Isolation of RNA and DNA fragments using diatomaceous earth." BIOTECHNOLOGY TECHNIQUES, vol. 12, no. 7, July 1998 (1998-07), pages 549-552, XP008021157 ISSN: 0951-208X
- LORENZ M G ET AL: "HIGHLY EFFICIENT GENETIC TRANSFORMATION OF BACILLUS-SUBTILIS ATTACHED TO SAND GRAINS" JOURNAL OF GENERAL MICROBIOLOGY, vol. 134, no. 1, 1988, pages 107-112, XP008021170 ISSN: 0022-1287
- LEVY M S ET AL: "Biochemical engineering approaches to the challenges of producing pure plasmid DNA" TRENDS IN BIOTECHNOLOGY, ELSEVIER, AMSTERDAM, NL, vol. 18, no. 7, 1 July 2000 (2000-07-01), pages 296-305, XP004213278 ISSN: 0167-7799

## Description

The invention relates to the use of multivalent cations and chelating agents for nucleic acid extraction and nucleic acid purification with silica-based supporting materials (supports), especially with clay minerals, sand and clay mineral/sand mixtures. Thus a universal method is provided for purifying nucleic acids from each kind of nucleic-acid containing material in any quantities.

Classically, in the state of the art, nucleic acids are released, by means of strongly denaturing and reducing agents, including hydrolytic enzymes (e.g. proteases, lysozyme, lyticase), from cells and tissues and subsequently extracted and purified with a mixture of chloroform and phenol. The nucleic acids are finally obtained from the aqueous phase by ethanol precipitation or narrowing down by means of dialysis (Sambrook, J., Russell, D.W. (2001): Molecular cloning - a laboratory manual. Cold Spring Harbor Laboratory Press, Cold Spring Harbor, New York).

These "classical" methods are especially time-consuming (sometimes taking up to 48 hours), require a considerable amount of equipment, and relatively large quantities of biological material and, in addition, involve a considerable health risk (amongst other things due to the use of choroform and phenol).

In the last few years, alternative extraction and purification methods have been developed, aimed at simplification and reduction of the health risk. These are based on the method of Vogelstein and Gillespie (Proc. Nat. Acad. Sci., USA 76, 1979, 615-619), which has for the first time been developed for the preparative and analytical extraction of DNA fragments from agarose gels. According to this method DNA-containing pieces of agarose are dissolved and the DNA reversibly bound, in the presence of high concentrations of chaotropic salts (sodium iodide or guanidine salts) to silica or silicates (glass beads or glass milk). In the further purification method, the silica matrix is washed with a buffer solution (20 mmol/l Tris-HCl, pH 7.2, 0.2 mol/l NaCl; 2 mmol/l EDTA; 50% ethanol) and the DNA finally eluted with water or dilute buffer solutions (e.g. 10 mmol/l Tris-HCl, pH 8.0).

Modified methods based on this method were used for the extraction and purification of nucleic acids from various materials (Marko, M.A., Chipperfield, R., Birnboim, H.G., Anal. Biochem. 121, 1982, 382-387), amongst other things plasmid DNA from bacterial lysates and genomic DNA and total RNA from blood, animal and human or plant tissues and cell cultures.

These methods, commercially marketed in the form of kits, e.g. from QIAGEN (Hilden, DE) or Macherey-Nagel (Düren, DE), are based on the principle that nucleic acids bind to mineral supports in the presence of high ionic strength, especially chaotropic salts. Finely ground glass powder (e.g. Promega; MoBio), diatomaceous earth (Sigma) or silica gels (Qiagen) have proved successful as supporting materials, for which chemically modified materials such as silica carbide (U.S. Pat. No. 6,291,248) can also be used.

U.S. Patent No. 5,234,809 discloses a method for the purification of nucleic acids from various materials, including blood, blood serum, faeces, urine and cell cultures, which has proved successful. In this method, a buffer with chaotropic agents (e.g. detergent and guadinine salt in a high concentration) is used for the cell lysis and at the same time for binding the nucleic acids to a solid support (e.g. silica, polystyrene balls, nitrocellulose paper). Adequately known washing steps with ethanol- or isopropanol-containing solutions are used, to remove dissolved impurities from the support. Elution with water or low-salt buffers such as 10 mmol/l Tris or TE (10 mmol/l Tris, 1 mmol/l EDTA) finally effects the desorption of the nucleic acids from the supporting material.

An advantage of this method is that chaotropic salts ensure the irreversible denaturing and thus inactivation of nucleases. Essential disadvantages of the method are that the concentration of the chaotropic salts to some extent have to be strongly adjusted to the material used, and also the lysis of biological material, such as fungal or plant tissue, is sometimes only very inefficient. Moreover, for the frequently selected use of enzymes (proteinase, RNase) in the purification methods, the concentration of chaotropic agents must be reduced below values which otherwise bring about inactivation of nucleases.

Furthermore, an alternative technology, based on silica, is described which does not involve the use of chaotropic salts (cf. DE 198 56 064 A1). An advantage of this commercially available method is that nucleic acids can be extracted even from materials with a very small nucleic acid content with a universal protocol. A disadvantage is that the nucleic acid preparations do not without exception meet the required quality standards (including photometric measurement), and thus have absorption ratios of less than 1.70 at 260 nm to 280 nm.

It was known in the art that certain clay minerals may be used for binding DNA. For example, binding of bacterial DNA to the clay mineral montmorillonite is reported in Khanna and Stotzky (1992), Appl. Environ. Microbiol., 58(6), 1930-9. Further, Lorenz and Wackernagel (1991), Appl. Environ. Microbiol., 57(4), 1246-1251 described the adsorption of DNA to sand.

WO 99/58664 describes the isolation of DNA by use of paramagnetic microparticles having a surface coated with functional groups, such as carboxyl groups. The DNA is bound to the microparticles in the presence of a MgCl₂-containing buffer, and the microparticles are subsequently washed with an alcohol-containing buffer.

A further method is based on the chromatographic purification of nucleic acids by means of exchange resins (U.S. Patent No. 5,057,426). In this method, the nucleic acids in cell lysates are bound to positively charged groups of the resin, and after various washing steps are eluted from the matrix by means of high anion concentrations. This method is especially used for the extraction and purification of nucleic acids from large quantities of biological material. A common commercial application uses gravitation-driven through-flow columns, which deliver high-purity nucleic acids. A disadvantage of the system is that the method is very time-consuming (more than 16 hours in the case of chromosomal DNA), as the nucleic acids have to be desalinated and precipitated following elution, to then be dissolved in water or a low-salt buffer (e.g. 10 mmol/l Tris-HCl, pH 8.0 or 10 mmol/l Tris, 1 mmol/l EDTA). In addition, the matrix may be clogged by the presence of extracellular, high-polymer substances (e.g. mucus), so that no nucleic acid, or only small quantities, or nucleic acid of reduced quality can be obtained.

It is therefore the object of the invention to make available a method by which high-purity DNA and total RNA (including tRNA, mRNA, rRNA, mitochondrial RNA and hnRNA) can be prepared, and which is universally applicable with regard to the nucleic-acid-containing source material, and is quick and simple to handle.

This object is solved according to the invention, by the use of buffers which contain multivalent cations.

The invention thus relates to the extraction and purification of nucleic acids using (1) silica-based supports, but especially mineral supporting materials, which hitherto have not yet been proposed and/or used as supporting material for the isolation of nucleic acids, in the presence of (2) new buffers for these supporting materials, which specifically cause nucleic acids to bind to the above-mentioned supports and especially to mineral supports, and which effect the removal of contaminating substances from the support and the elution of nucleic acids from the support.

### Re. (1) :

In the method according to the invention, silica-based supports, such as, for example, glass-fibre non-woven fabrics (fleeces) or silicon compounds of different particle sizes can be used as supporting material.

Especially preferably, use is made of clay minerals, sand or mixtures of clay mineral and sand (clay mineral-sand mixtures), which differ distinctly from the silica materials used hitherto for nucleic acid purification in their diagenesis, mineralogy and physico-chemical properties.

Clay minerals are minerals belonging predominantly to the phyllosilicates, but in some cases also to the band silicates (e.g. palygorskite (attapulgite) and sepiolite (meerschaum), which form the main mineral stock of the clays and clays stones, and also occur in silts and silt stones, clay slates and some sands and sandstones. Clay minerals which consist of series of 1 tetrahedron and 1 octahedron layer each are called two-layer clay minerals or 1:1 minerals, or also 7 Å clay minerals after the spacing, referred to in the specialist terminology as base spacing, of the tetrahedron layers; these include e.g. kaolinite, dickite and nakrite. Clay minerals from formations of 1 octahedron and 2 tetrahedron layers are called three-layer or 10 Å minerals or 2:1 minerals; these include illite, the smectites (montmorillonite is the main representative of the smectite group and the main component of bentonite. In practice bentonite, smectite and montmorillonite are used as synonyms for multi-layer silicates which may be used as sources.), glauconite and vermiculite. If a further independent octahedron layer is incorporated between the three-layer formations, four-layer or 14 Å minerals are produced; examples are the chlorites. A special clay mineral group is represented by interbedded minerals. Between the layer packages, which can be moved against each other relatively easily, and produce the laminated structure which is typical of many clay minerals and also explains their perfect cleavability, ions and water molecules can, for example, become embedded; this can lead to an expansion of the layer spacings (swelling) e.g. in the case of the smectites.

The preferred subject of the invention is thus the use of clay minerals for isolation of nucleic acids from nucleic-acid-containing material and/or for purification of nucleic acids, wherein the clay mineral within the meaning of the invention is an individual clay mineral or a mixture of different clay minerals, preferably a 1:1 and/or 2:1 clay mineral. According to a particular embodiment, kaolinite and/or montmorillonite (bentonite) is used. Whenever mention is made of "a clay mineral" below, this term is also intended to include the abovementioned mixture.

Geologically, sand is a mixture of small, approximately round fragments/particles, which are produced during the disintegration of rocks. The diameter of the particles is between 0.05 and 2 mm. Sand consists mainly of quartz, and in addition contains other minerals such as silt (very fine sand, 0.002 to 0.06 mm in diameter), mica, feldspar, calcite (CaCO₃), magnetite, clay minerals and heavy minerals.

The subject of the invention is thus also the use of sand for the isolation of nucleic acids from nucleic-acid-containing material and/or for the purification of nucleic acids. Within the meaning of the invention, this may include sands of different origin and also artificial mixtures of sand and minerals.

According to a particular embodiment, use is made of acid-treated and calcined sea sand (Merck) or mixtures of the above-mentioned sea sand and clay minerals, in particular kaolinite and/or montmorillonite (bentonite), e.g. sea sand and clay mineral in the ratio 100:1. Whenever mention is made of "sand" below, this term is also intended to include the above-mentioned mixtures.

### Re. (2) :

According to the invention, buffers are used, which lead to the binding, purification and elution of nucleic acids onto the above-mentioned silica-based supports, in particular onto the clay minerals and/or sand. For the method according to the invention, the presence of multivalent, but not monovalent, cations, in a low concentration (≤ 0.2 mol/l) is sufficient for the binding of nucleic acids to mineral supports. Within the meaning of the invention, for the purification and elution of nucleic acids, it is obligatory to use buffers which contain chelating agents and, for purification, alcohols.

The subject of the invention is thus the use of a buffer, which contains a salt of one or more (ie. at least one) multivalent cation(s), to bind nucleic acids to silica-based supports, in particular to clay minerals, sand or clay mineral/sand mixtures.

The invention relates in particular to a method or process for the isolation and/or purification of nucleic acids, in which nucleic-acid-containing material as a sample
a) is brought into contact with a supporting material in the presence of a salt of a multivalent cation for the adsorption of nucleic acids, wherein said supporting material is a clay mineral, sand or a mixture of clay mineral and sand, or a silica-based support or a silicon compound;
b) the supporting material is washed at least once with a buffer containing an alcohol and a chelating agent, and
c) the nucleic acids are isolated, by adding an aqueous solution of a chelating agent suitable for the cation to the supporting material for desorption of the nucleic acids, and separating the aqueous, nucleic-acid-containing solution from the supporting material.

Optionally, prior to step (b) the support and sample are separated from each other (step a').

In the method, in stage (a), several salts of a multivalent cation or of several multivalent cations can be used. These cations are preferably Mg²⁺, Ca²⁺, Mn²⁺ and/or Al³⁺ and the salts are in particular MgCl₂, CaCl₂, MnCl₂, and/or AlCl₃. Correspondingly, in stages (b) and (c) several chelating agents can be used at the same time.

The method can be implemented by producing the support in the form of a suspension in a suitable buffer. Alternatively, the method can be implemented in the form of a spin column, which is filled with the supporting material (preferably sand or a mixture of sand and clay mineral) (and a buffer solution is preferably also added to this).

In the last-mentioned spin-column variant of the method, steps (a) and (a') can be carried out by applying the nucleic-acid-containing material (sample) to the spin column and carrying out centrifugation.

Step (b) can be carried out by applying a washing solution to the spin column and carrying out centrifugation, and possibly repeating this step several times, possibly with different washing solutions. According to a particular embodiment, step (b) is carried out several times, using an EDTA-containing buffer solution, which further contains an alcohol. However, it is also possible to wash first with an alcoholic buffer solution and then with an EDTA-containing buffer solution which contains an alcohol. The alcohol used is preferably ethanol, propanol and/or isopropanol.

The invention further relates to a variant of the method, in which treatment with one or more RNases or DNases is carried out before step (a), or after step (a) or (a') and before step (c).

A further subject of the invention is a kit for carrying out the method or the above-mentioned method variants according to the invention. The kit preferably contains either a suspension of clay mineral(s) in a suitable buffer or a spin column filled with sand or a mixture of sand and clay mineral. It is further advantageous if the kit also contains agents for cell lysis, washing solutions (washing buffer) and/or other equipment, means or reagents necessary to carry out the method. According to a particular embodiment the kit contains the following buffers and solutions:
RS (10 mmol/l EDTA, 50 mmol/l Tris-HCl, pH 8.0), CH (5 mol/l guanidine-isothiocyanate, 5% Tween-20), AB (0.3 mol/l MgCl₂, 0.3 mol/l Tris-HCl, pH 9.5, 20% isopropanol), AL1 (0.2 mol/l NaOH, 1% SDS), AL2 (0.2 mol/l MgCl₂, 0.8 mol/l Tris-HCl, pH 9.5), WB (0.2 mol/l EDTA, 50 mmol/l Tris-HCl, pH 9.5, 40% isopropanol), TE (10mmol/l Tris-HCl, pH 8.0, 1 mmol/l EDTA).

It has surprisingly been found that, according to a particular embodiment of the invention, nucleic acids (DNA and total RNA) of the highest quality can be isolated from nucleic-acid-containing material, using cell-lysing agents
- with buffered suspension of the supporting materials, in particular of the clay minerals, in the presence of low concentrations (≤ 0.2 mol/l) of salts of multivalent, but not of monovalent, cations (0.6 mol/l) in the compound,
- with chelating agents in a washing step and subsequent known washing steps with alcohol-containing solutions (e.g. ethanol or isopropanol)
- and with chelating agents for the desorption of the nucleic acids from the supporting material, but not, on the other hand, as known from the state of the art, in other purification methods, using silicate supports with water or dilute buffer (e.g. 10 mmol/l Tris-HCl, pH 8.0).

Essential to purification and subsequent elution is a washing step with a buffer which contains, e.g. EDTA (0.2 mol/l) and an alcohol (e.g. 40% isopropanol). On the other hand, for elution, buffers (10 mmol/l Tris, pH 8.0) with lower EDTA concentrations are required (1 to 100 mmol/l). For elution, in some applications, the use of heated buffers (e.g. 70°C) and/or buffers with increased pH values (e.g. 10 mmol/l Tris, pH 9.5) have proved advantageous for increased nucleic acid yields.

The presence of chaotropic salts (e.g. guadinine salts) in high concentrations is not obligatory for the resultant binding of the nucleic acids to the supports within the meaning of the invention. Further, concentrations of salts such as NaCl, KC1 or K-acetate, chaotropic salts such as guanidine hydrochloride or guanidine isothiocyanate, other chaotropic agents (e.g. detergents), low concentrations of chelating agents (e.g. ethylene diamine tetraacetic acid, EDTA; bis-aminoethyl glycoether-N,N,N',N'-tetraacetic acid, EGTA) and/or protein-disintegrating enzymes (e.g. proteinase K) do not interfere with the adsorption of the nucleic acids onto the supporting materials within the meaning of the invention.

It is also surprising that - in contrast to methods using glass and silica materials in the state of the art - the binding of nucleic acids to mineral supports within the meaning of the invention, in a particular embodiment clay minerals and sand, is independent of the pH value of the nucleic-acid-containing solution.

The nucleic-acid-containing solutions can be obtained by incubation of nucleic-acid-containing materials with lysis buffers, which contain either (1) chaotropic salts such as guadinine salts, (2) alkaline compounds such as NaOH, (3) neutral, anionic or cationic detergents such as sodium dodecyl sulphate (SDS), TritonX-100, TWEEN-20 or hexadecyl trimethyl ammonium bromide CTAB or (4) enzymes such as lysozyme in bacteria, lyticase in yeasts, chitinase in fungi, or proteases in tissues.

The following can, for example, be used as nucleic-acid-containing materials for the production of nucleic-acid-containing solutions: viruses, bacteriophages, cell material (in particular cell fragments from cell decomposition, e.g. of bacteria) for the extraction of plasmids and vectors (including cosmids, phagemids, "bacterial artificial chromosomes" [BACs], "yeast artificial chromosomes" [YACs], "P1-derived artificial chromosomes" [PACs]), prokaryotes (eubacteria, archaea), yeasts, lower and higher fungi, plant material (including fruit, leaves and stems), invertebrates such as snails, blood and tissue of humans and animals, human, animal and plant cell cultures, urine, faeces, foodstuff (including fish, milk, sausage, preserved food), forensic specimen material, earth (soil) and material such as nucleic-acid-containing agarose gels or PCR reaction mixtures, in order to extract nucleic acids or nucleic acid fragments.

The term "nucleic acids" below is intended to mean DNA and/or total RNA. The term "total RNA" covers the RNA species occurring in a cell, including tRNA, mRNA, rRNA, mitochondrial RNA and hnRNA.

For the binding of nucleic acids to the above-mentioned silica-based supporting materials, in particular to clay minerals and sand, low concentrations of salts of multivalent cations (e.g. Mg²⁺, Ca²⁺, Mn²⁺, or A1³⁺, preferably MgCl₂, CaCl₂, MnCl₂ and/or AlCl₃ (e.g. MgCl₂ < 0.08 mol/l) are already sufficient. These can for example be magnesium chloride, calcium chloride, manganese chloride and/or aluminium chloride-containing buffers.

Furthermore, EDTA- or EGTA-containing solutions can for example be considered as washing solutions which contain a chelating agent.

Preferred clay minerals are "two-layer clay minerals", also known as 1:1 clay minerals, and "three-layer clay minerals", also known as 2:1 clay minerals, such as, for example, kaolinite and/or montmorillonite (bentonite).

To optimise the binding and subsequent purification method of the nucleic acids to the above-mentioned supporting materials, in particular to the clay materials and sand, according to a preferred embodiment of the invention, an alcoholic component, such as isopropanol or ethanol and/or mixtures thereof, is also added.

Following short-term contact/incubation of the lysate with the supporting material, the lysate is separated from the supports, e.g. via a short centrifugation step. In the further purification method, the nucleic acid-carrying support is washed with an EDTA-containing washing buffer, which additionally contains an alcoholic component such as, e.g. 40% (v/v) ethanol or 40% isopropanol. Subsequently, washing is carried out with 70% alcohol in a known manner. The supporting material is dried, and the adsorbed nucleic acid is dissolved with chelating agent-containing buffer. The possibility exists, depending on whether DNA or total RNA is to be isolated, of inserting an enzymatic (reaction) step by the use of RNases (e.g. RNase A) or DNases (e.g. DNase I). To do this, following the separation of the lysate from the support, washing is first carried out with an alcohol-containing solution, e.g. 70% ethanol, before enzymatic digestion is carried out. Subsequently, washing is carried out with an EDTA- and alcohol-containing washing buffer and the method is continued as described above.

Surprisingly, within the framework of the invention, it emerged that for the desorption of nucleic acids, low concentrations of a chelating agent suitable for the cation (or for its chelation, respectively) i.e. a chelating compound such as, e.g. EDTA, are required. For example, 1 to 100 mmol/l EDTA is suitable. In contrast, distilled water or buffer solutions such as 10 mmol/l Tris-HCl which are normally used to dissolve nucleic acids from glass or silica supporting materials produced only a minor detachment, or no detachment at all, of nucleic acid bound to the supporting material.

The invention further concerns a kit for carrying out the purification/isolation method according to the invention, which contains the solutions and/or reagents and supporting materials required for nucleic acid purification, in particular clay minerals and/or sand (preferably already suspended in suitable buffer), which make it possible to subject a nucleic-acid-containing material to the method described.

The kit preferably contains the following solutions which are needed for the purification of nucleic acids according to the invention, such as (a) magnesium chloride-, calcium chloride-, manganese chloride- and/or aluminimum chloride-containing buffer, (b) EDTA or EGTA-containing buffer,(c) ethanol- or isopropanol-containing buffer, (d) DNase, or RNase A or other nuclease(s)- and/or protease(s)-containing solution. These solutions can already be available ready-for-use, or can be freshly prepared as required.

According to a particular embodiment, the kit can contain solutions for lysis of nucleic-acid-containing material and the clay mineral or minerals with sand, or sand alone as a spin column version.

### Summary - Advantages of the System

Within the framework of this invention, it has been shown for the first time that clay minerals and sand are highly suitable as supporting materials for the binding and purification of nucleic acids from various nucleic-acid-containing material. For the binding of the nucleic acids to the support, only a very low concentration (≤ 0.2 mol/l) of salts of multivalent cations is required, in which the adsorption method, in contrast to the state of the art, takes place independently of the pH of the solution. With buffers of the above-mentioned multivalent cations, good results can also be achieved using other silica-based supporting materials.

Also contrary to the state of the art with glass or silica materials (DE 198 56 064 Al), the binding of nucleic acids to clay minerals is not achieved in the presence of salts of monovalent cations (e.g. ≤ 0.6 mol/l Na⁺ or K⁺). Within the meaning of the invention and contrary to the state of the art, the use of chelating-agent-containing buffers is necessary for the purification and elution of the nucleic acids from the supporting material.

The invention has the advantage that a new and universally applicable method for the production of pure nucleic acids from various nucleic-acid-containing materials has been developed.

The method contains a uniform protocol for the lysis of the material, as high concentrations of chaotropic agents, chelating chemicals and the pH values of solutions exert no negative influence on the binding of nucleic acids to the supporting material. Thus the usual lysis methods, e.g. the use of alkali with high pH values, high detergent and guanidine salt concentrations, can be directly tied into the method according to the invention for the purification of nucleic acids from various nucleic-acid-containing materials, further underlining the universality of the system.

A further advantage of the method is that both DNA and RNA can be adsorbed and again dissolved under the conditions according to the invention, whereby the simultaneous purification of both nucleic acid species and/or the differential purification of DNA or RNA can be achieved by the use of RNases/DNases in a universal method. The method according to the invention is in addition quick and simple and delivers high-quality nucleic acid preparations which are eminently suitable for subsequent processes such as PCR, hybridisations, restrictions or ligations.

The invention is explained below with reference to examples.

### Examples

### Examples of application:

Buffers and solutions used: RS (10 mmol/l EDTA, 50 mmol/l Tris-HCl, pH 8.0), CH (5 mol/l guanidine-isothiocyanate, 5% Tween-20), AB (0.3 mol/l MgCl₂, 0.3 mol/l Tris-HCl, pH 9.5, 20% isopropanol), AL1 (0.2 mol/l NaOH, 1% SDS) , AL2 (0.2 mol/l MgCl₂, 0.8 mol/l Tris-HCl, pH 9.5), WB (0.2 mol/l EDTA, 50 mmol/l Tris-HCl, pH 9.5, 40% isopropanol), TE (10 mmol/l Tris-HCl, pH 8.0, 1 mmol/l EDTA).

### Example 1

### Isolation of genomic DNA from microorganisms

### A) Eubacteria and archaea

Cell lysis: Source materials are stationary liquid cultures (1 - 1.5 ml) of various bacteria and halobacteria. To obtain the cells, bacteria/halobacteria suspensions were centrifuged off and the supernatant quantitatively removed. The cells were resuspended in 50 µl RS buffer possibly including 10 mg/ml lysozyme (eubacteria) and possibly incubated at 37°C for 15 minutes. Cell lysis was achieved by adding 250 µl CH and brief vortexing. The lysate was mixed with 200 µl AB by vortexing and immediately placed in a spin column (e.g. commercial spin column from AHN, Nordhausen), which contained a sand-bentonite mixture (0.5 g sand, ca. 5 mg bentonite).

Washing steps: The column was then immediately centrifuged at ≥ 16,000 x g for 15 seconds. The column was then washed with 0.5 ml WB, then twice consecutively by the application of 0.5 ml 70% (v/v) EtOH by centrifugation for 30 seconds in each case.

DNA elution: 100 - 150 µl TE were added to the washed column and incubated for 15 min. The DNA was transferred to a 1.5 ml polypropylene reaction vessel by 0.5 - 1 min centrifugation.

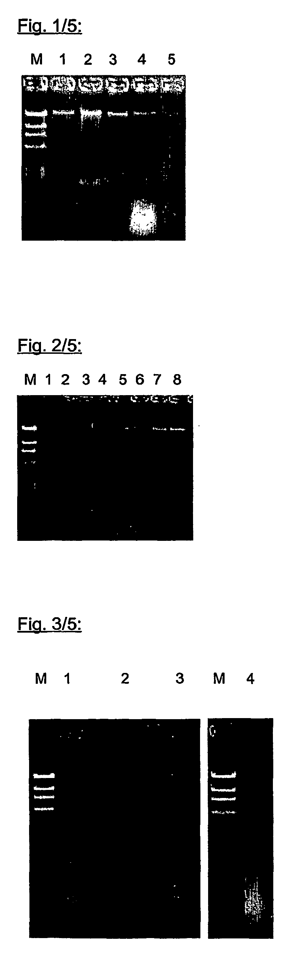

For result see Fig. 1.

### B) Yeasts

1 - 2 ml stationary yeast culture were centrifuged at ≥ 16,000 x g, the sedimented cells were resuspended in 1 ml 1 M saccharose, 0.1 mol/l EDTA, 14 mmol/l β-mercaptoethanol and 100 U lyticase and incubated at 30°C for 30-45 minutes. The spheroplasts were centrifuged off at 10,000 x g for 10 min, the supernatant was decanted and then lysed in 250 µl CH with powerful vortices and subsequently mixed with 200 µl AB. After application to a spin column the method was continued as under 1. A).

For result see Figures 1 and 2.

### Example 2

### Isolation of genomic DNA and total RNA from plants and snails

Fresh plant material (e.g. leaves, seeds, shoots) or several deep-frozen whole snails including shells were pounded under liquid nitrogen, and 50 - 100 mg of the powder transferred to a 1.5 ml polypropylene reaction vessel.

After the addition of 0.6 ml CH and extensive vortexing ( 15 - 30 s), incubation was carried out at 65°C for 30 minutes, with vortexing at 10-minute intervals. The lysate was centrifuged for 5 minutes at ca. 10,000 x g, and 300 µl of the supernatant were mixed with 300 µl AB. The further protocol corresponds to that from Example 1. A).

For result see Figure 3.

### Example 3

### Isolation of genomic DNA from full blood

100 µl full blood, with 0.1% EDTA added as coagulation inhibitor, were centrifuged at ca. 18,000 x g for 30 seconds and the plasma supernatant removed. For lysis of the cells, the sediment was mixed with 250 µl CH by vortexing, and then mixed with 200 µl AB. A spin column was charged with the lysate and centrifuged for 30 s, then charged with 0.5 ml WB and incubated for 15 min. Washing was then carried out twice with 70% EtOH. Elution took place with TE heated to 70°C, followed by incubation for 15 min and centrifugation for 30 s.

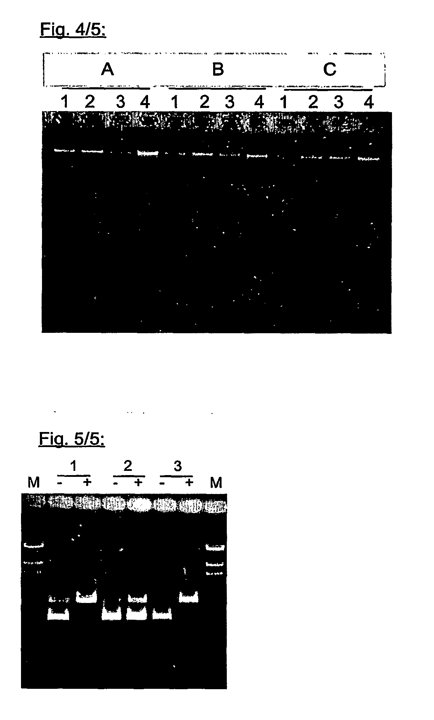

For result see Figure 4.

### Example 4

### Isolation of plasmid DNA

A 1.5 ml stationary E. coli culture was centrifuged at ca. 18,000 x g for 30 seconds, the supernatant was quantitatively decanted, and the cell sediment was resuspended in 30 µl RS incl. 100 µg/ml RNase A by vortexing. After the addition of 30 µl AL1, mixing was carried out carefully with the pipette tip, until lysis occurred. Then 30 µl ice-cold AL2 were mixed with the lysate as described above, centrifuged at ca. 18,000 x g and 4°C for 15 min and the supernatant mixed with 90 µl AB. The lysate was then placed in a spin column and incubated for 15 min. After centrifugation for 30 s, the method was continued as under 1. A) "washing steps" and "DNA elution".

For result see Figure 5.

### Figures:

- Fig. 1:: Gel electrophoretic representation of the isolation of genomic DNA from 1 - 2 ml stationary cultures of various microorganisms (0.8% TAE agarose gel, stained with ethidium bromide).
Samples 1 - 5 (proportion of the DNA preparation applied):
1: Halobacterium sp., Archaea (1/50)
2: Pseudomonas stutzeri, gram-negative eubacterium (1/75)
3: Bacillus subtilis, gram-positive eubacterium (1/50)
4: Saccharoymyces carlsbergensis, yeast (1/150)
5: Aspergillus niger, fungus (1/75)
- Fig. 2: Gel electrophoretic representation of the isolation of genomic DNA and total RNA from 2 ml yeast culture (Pichia pastoris Gas115) (0.8% TAE agarose gel, stained with ethidium bromide); 1/50 of the DNA preparation was applied.
Samples 1 - 8: Isolation of nucleic acids from various growth phases:
1: 20 h; 2. 22 h; 3: 25 h, 4: 27 h; 5: 29 h; 6: 31 h; 7: 44 h, 8: 46 h
M: molecular size marker (Lambda DNA, HindIII restricted): 23.1 kb; 9.4 kb; 6.5 kb; 4.4 kb; 2.3 kb; 2.0 kb; 0.6 kb (from top to bottom).
- Fig. 3: Gel electrophoretic representation of the isolation of genomic DNA and total RNA from 50 mg plant material/snail (0.8% TAE agarose gel, stained with ethidium bromide); 1/10 of the preparation was applied.
Sample 1: Honesty, leaf (Lunaria annua)
Sample 2: Ivy, leaf (Hedera helix)
Sample 3: Yellow archangel, leaf (Galeobdolon argentatum)
Sample 4: Snail (Cepaea sp.)
M: molecular size marker (Lambda DNA, Hind III restricted): 23.1 kb; 9.4 kb; 6.5 kb; 4.4 kb; 2.3 kb; 2.0 kb; 0.6 kb (from top to bottom).
- Fig. 4:: Representation of the gel electrophoresis of purified genomic DNA from 100 µl full blood (0.8% TAE agarose gel, stained with ethidium bromide); 1/15 of the DNA preparation was applied.
Samples 1-4: DNA isolated from various healthy individuals. A-C: Repeat tests.
- Fig. 5:: Isolation of plasmid-DNA (pUC13) according to the procedure of the invention with a spin column, compared with commercial kits from two suppliers (agarose gel electrophoresis, 0.8% gel, stained with ethidium bromide); in each case 1/20 of the plasmid-DNA preparation was applied.
Samples 1-2: Plasmid-DNA extraction by means of commercial plasmid-DNA extraction kits from two manufacturers
   3: Plasmid-DNA extraction by means of the method according to the invention
-/+ without/with EcoRI-digestion.
M: molecular size marker (Lambda DNA, HindIII restricted): 23.1 kb; 9.4 kb; 6.5 kb; 4.4 kb; 2.3 kb; 2.0 kb; 0.6 kb (from top to bottom).

## Claims

1. Method for the isolation and/or purification of nucleic acids, **characterised in that** nucleic-acid-containing material as a sample
a) is brought into contact with a supporting material in the presence of a salt of a multivalent cation for the adsorption of nucleic acids, wherein said supporting material is a clay mineral, sand or a mixture of clay mineral and sand, or a silica-based support or a silicon compound;
b) the supporting material is washed at least once with a buffer containing an alcohol and a chelating agent, and
c) the nucleic acids are isolated, by adding an aqueous solution of a chelating agent suitable for the cation to the supporting material for desorption of the nucleic acids, and separating the aqueous, nucleic-acid-containing solution from the supporting material.

2. Method according to claim 1, **characterised in that** prior to step (b), the supporting material and the sample are separated from each other (step a').

3. Method according to claim 1 or 2, **characterised in that** the clay mineral is a mixture of clay minerals.

4. Method according to any of claims 1-3, **characterised in that** the clay mineral is a 1:1 clay mineral and/or 2:1 clay mineral.

5. Method according to claim 1, **characterised in that** the clay mineral is kaolinite and/or montmorillonite (bentonite).

6. Method according to claims 1 to 5, **characterised in that** the nucleic-acid-containing material is chosen from the group consisting of viruses, bacteriophages, intact cells, cell fragments, prokaryotes, yeasts, lower and higher fungi, plant material, invertebrates, blood and tissue from humans and animals, human, animal and plant cell cultures, urine, faeces, foodstuff, forensic specimen material, earth, nucleic-acid-containing agarose gels or PCR reaction mixtures.

7. Method according to claims 1 to 6, **characterised in that** in step (a) several salts of a multivalent cation or several multivalent cations are used.

8. Method according to claims 1 to 7, **characterised in that** the salt is MgCl₂, CaCl₂, MnCl₂, and/or AlCl₃.

9. Method according to claims 1 to 8, **characterised in that** in step (c) several chelating agents are used.

10. Method according to claims 1 to 9, **characterised in that** the supporting material is provided in the form of a suspension in a suitable buffer.

11. Method according to claims 1 to 9, **characterised in that** the supporting material is provided in the form of a spin column.

12. Method according to claim 11, **characterised in that** steps (a) and (a') are carried out by applying the nucleic-acid-containing material (sample) to the spin column and carrying out centrifugation.

13. Method according to claims 11 and 12, **characterised in that** step (b) is carried out by applying a washing solution to the spin column and carrying out centrifugation, and repeating this step several times, possibly with different washing solutions.

14. Method according to claim 13, **characterised in that** step (b) is carried out several times, using an EDTA-containing buffer solution for washing, which further contains an alcohol.

15. Method according to claim 13, **characterised in that** washing is first carried out with an alcoholic buffer solution and then with an EDTA-containing buffer solution which contains an alcohol.

16. Method according to claim 14 and 15, **characterised in that** the alcohol is ethanol, propanol and/or isopropanol.

17. Method according to claims 1 to 12, **characterized in that** treatment with one or more RNases or DNases is carried out before step (a), or after step (a) or (a') and before step (c).

## Patentansprüche

1. Verfahren zur Isolierung und/oder Aufreinigung von Nukleinsäuren, bei dem Nukleinsäure-haltiges Material als Probe
a) für die Adsorption von Nukleinsäuren in Gegenwart eines Salzes eines multivalenten Kations mit einem Trägermaterial in Kontakt gebracht wird, wobei das Trägermaterial ein Tonmineral, Sand oder eine Mischung aus Tonmineral und Sand oder ein auf Silica basierender Träger oder eine Siliciumverbindung ist;
b) das Trägermaterial mindestens einmal mit einem Puffer gewaschen wird, der einen Alkohol und einen chelatierendes Mittel umfasst, und
c) die Nukleinsäuren isoliert werden, indem eine wässrige Lösung eines chelatierenden Mittels, das für das Kation geeignet ist, für die Desorption der Nukleinsäuren zu dem Trägermaterial gegeben wird, und die wässrige Nukleinsäure-haltige Lösung von dem Trägermaterial getrennt wird.

2. Verfahren nach Anspruch 1, bei dem vor Schritt (b) das Trägermaterial und die Probe voneinander getrennt werden (Schritt a').

3. Verfahren nach Anspruch 1 oder 2, bei dem das Tonmineral eine Mischung von Tonmineralien ist.

4. Verfahren nach einem der Ansprüche 1 bis 3, bei dem das Tonmineral ein 1:1 Tonmineral und/oder ein 2:1 Tonmineral ist.

5. Verfahren nach Anspruch 1, bei dem das Tonmineral ein Kaolinit und/oder Montmorillonit (Bentonit) ist.

6. Verfahren nach den Ansprüchen 1 bis 5, bei dem das Nukleinsäure-haltige Material ausgewählt ist aus der Gruppe bestehend aus Viren, Bakteriophagen, intakten Zellen, Zellfragmenten, Prokaryonten, Hefen, niederen und höheren Pilzen, Pflanzenmaterial, Invertebraten, Blut und Gewebe von Menschen und Tieren, humanen, tierischen und pflanzlichen Zellkulturen, Urin, Fäkalien, Nahrungsmittel, forensisches Probenmaterial, Erde, Nukleinsäure-haltige Agarosegele oder PCR-Reaktionsmischungen.

7. Verfahren nach den Ansprüchen 1 bis 6, bei dem in Schritt (a) mehrere Salze eines multivalenten Kations oder mehrere multivalente Kationen verwendet werden.

8. Verfahren nach den Ansprüchen 1 bis 7, bei dem das Salz MgCl₂, CaCl₂, MaCl₂ und/oder AlCl₃, ist.

9. Verfahren nach den Ansprüchen 1 bis 8, bei dem in Schritt (c) mehrere chelatierende Mittel verwendet werden.

10. Verfahren nach den Ansprüchen 1 bis 9, bei dem das Trägermaterial in Form einer Suspension in einem geeigneten Puffer bereitgestellt wird.

11. Verfahren nach den Ansprüchen 1 bis 9, bei dem das Trägermaterial in Form einer Spinsäule bereitgestellt wird.

12. Verfahren nach Anspruch 11, bei dem die Schritte (a) und (a') durchgeführt werden, indem das Nukleinsäure-haltige Material (Probe) auf die Spinsäule aufgetragen wird und eine Zentrifugation durchgeführt wird.

13. Verfahren nach den Ansprüchen 11 bis 12, bei dem Schritt (b) durchgeführt wird, indem eine Waschlösung auf die Spinsäule aufgetragen wird und eine Zentrifugation ausgeführt wird, und dieser Schritt einige Male wiederholt wird, möglicherweise mit verschiedenen Waschlösungen.

14. Verfahren nach Anspruch 13, bei dem Schritt (b) mehrfach durchgeführt wird, wobei eine EDTA-enthaltende Pufferlösung für das Waschen verwendet wird, die ferner einen Alkohol umfasst.

15. Verfahren nach Anspruch 13, bei dem das Waschen zunächst mit einer alkoholischen Pufferlösung durchgeführt wird und anschließend mit einer EDTA-enthaltenden Pufferlösung, die einen Alkohol umfasst.

16. Verfahren nach Anspruch 14 und 15, bei dem der Alkohol Ethanol, Propanol und/oder Isopropanol ist.

17. Verfahren nach den Ansprüchen 1 bis 12, bei dem die Behandlung mit einer oder mit mehreren RNasen oder DNasen vor Schritt (a) oder nach Schritt (a) oder (a') und vor Schritt (c) durchgeführt wird.

## Revendications

1. Procédé d'isolement et/ou de purification d'acides nucléiques, **caractérisé en ce que** le matériau contenant des acides nucléiques utilisé comme échantillon
a) est amené en contact avec un matériau de support en présence d'un sel d'un cation polyvalent pour l'adsorption d'acides nucléiques, ledit matériau de support étant un minéral argileux, du sable, ou un mélange de minéral argileux et de sable, ou un support à base de silice ou un composé de silicium ;
b) le matériau de support est lavé au moins une fois avec un tampon contenant un alcool et un agent chélatant, et
c) les acides nucléiques sont isolés par addition, au matériau de support, d'une solution aqueuse d'un agent chélatant du cation, pour la désorption des acides nucléiques, et par séparation de la solution aqueuse contenant des acides nucléiques du matériau de support.

2. Procédé selon la revendication 1, **caractérisé en ce qu'**avant l'étape (b), le matériau de support et l'échantillon sont séparés l'un de l'autre (étape a').

3. Procédé selon la revendication 1 ou selon la revendication 2, **caractérisé en ce que** le minéral argileux est un mélange de minéraux argileux.

4. Procédé selon l'une quelconque des revendications 1 à 3, **caractérisé en ce que** le minéral argileux est un minéral argileux 1:1 et/ou un minéral argileux 2:1.

5. Procédé selon la revendication 1, **caractérisé en ce que** le minéral argileux est la kaolinite et/ou la montmorillonite (bentonite).

6. Procédé selon l'une quelconque des revendications 1 à 5, **caractérisé en ce que** le matériau contenant des acides nucléiques est choisi dans le groupe formé par les virus, les bactériophages, les cellules intactes, les fragments de cellules, les procaryotes, les levures, les champignons inférieurs et supérieurs, les matières végétales, les invertébrés, du sang et du tissu humains et animaux, des cultures de cellules humaines, animales et végétales, de l'urine, des matières fécales, des aliments, des matériaux d'échantillons médico-légaux, de la terre, des gels d'agarose contenant des acides nucléiques ou des mélanges de réactifs pour l'amplification en chaîne par polymérase (PCR).

7. Procédé selon l'une quelconque des revendications 1 à 6, **caractérisé en ce qu'**à l'étape (a) plusieurs sels d'un cation polyvalent ou plusieurs cations polyvalents sont utilisés.

8. Procédé selon l'une quelconque des revendications 1 à 7, **caractérisé en ce que** le sel est MgCl₂, CaCl₂, MnCl₂ et/ou AlCl₃.

9. Procédé selon l'une quelconque des revendications 1 à 8, **caractérisé en ce qu'**à l'étape (c) plusieurs agents chélatants sont utilisés.

10. Procédé selon l'une quelconque des revendications 1 à 9, **caractérisé en ce que** le matériau de support est fourni sous la forme d'une suspension dans un tampon adéquat.

11. Procédé selon l'une quelconque des revendications 1 à 9, **caractérisé en ce que** le matériau de support est fourni sous la forme d'une colonne centrifuge.

12. Procédé selon la revendication 11, **caractérisé en ce que** les étapes (a) et (a') sont réalisées en appliquant le matériau contenant les acides nucléiques (échantillon) sur la colonne centrifuge et en réalisant une centrifugation.

13. Procédé selon la revendication 11 et selon la revendication 12, **caractérisé en ce que** l'étape (b) est réalisée en appliquant une solution de lavage à la colonne centrifuge et en réalisant une centrifugation, et en répétant cette étape plusieurs fois, éventuellement avec différentes solutions de lavage.

14. Procédé selon la revendication 13, **caractérisé en ce que** l'étape (b) est réalisée plusieurs fois, en utilisant pour le lavage une solution tampon contenant de l'acide éthylène-diamine-tétra-acétique (EDTA) qui contient en outre un alcool.

15. Procédé selon la revendication 13, **caractérisé en ce que** le lavage est tout d'abord réalisé avec une solution tampon alcoolique et ensuite avec une solution tampon contenant de l'EDTA qui contient un alcool.

16. Procédé selon les revendications 14 et 15, **caractérisé en ce que** l'alcool est l'éthanol, le propanol et/ou l'isopropanol.

17. Procédé selon l'une quelconque des revendications 1 à 12, **caractérisé en ce que** le traitement avec une ou plusieurs ribonucléases ou désoxyribonucléases est réalisé avant l'étape (a), ou après l'étape (a) ou l'étape (a') et avant l'étape (c).
